# FASCICULE DE BREVET EUROPEEN

(11) **EP 1 092 422 B1**
(45) Date de publication et mention de la délivrance du brevet: **26.06.2002**
(21) Numéro de dépôt: 00118604.8
(22) Date de dépôt: 28.08.2000
(51) Int. Cl.: A61K 7/48, A61K 7/42

(54) **Composition, notamment cosmétique, comprenant une sapogénine**
Mittel insbesondere für Kosmetika enthaltend eine Sapogenin
Composition, particularly for cosmetics containing a sapogenin

(30) Priorité: 14.10.1999 FR 9912828
(43) Date de publication de la demande: 18.04.2001
(73) Titulaire: L'OREAL, 75008 Paris (FR)
(72) Inventeur: Rubinstenn, Gilles, 75013 Paris (FR); Baldo, Francine, 92330 Sceaux (FR); Guiramand, Carole, 91310 Linas (FR); Dreher, Susanne, 75020 Paris (FR)
(74) Mandataire: Renard, Emmanuelle

(56) Documents cités:
- EP-A- 0 358 970
- DE-A- 19 631 792
- FR-A- 2 466 273
- FR-A- 2 659 556
- GB-A- 2 080 142

## Description

La présente invention concerne une composition comprenant, dans un milieu physiologiquement acceptable, au moins une sapogénine.

Les sapogénines sont des composés résultant de l'hydrolyse acide des saponosides, qui sont des hétérosides de poids moléculaire très élevé présents dans le règne végétal.

Parmi les sapogénines, on citera en particulier : la diosgénine, l'hécogénine, la smilagénine, la sarsapogénine, la tigogénine, la yamogénine et la yuccagénine.

Ces composés ont en commun une structure stéroïde comprenant un nombre de substituants hydroxyle et/ou oxo variable et/ou un nombre variable de double liaisons. Ils sont connus comme précurseurs chimiques naturels d'hormones stéroïdiennes et décrits, à ce titre, comme constituants de choix de diverses préparations cosmétiques ou pharmaceutiques. Le document US-5,827,884 enseigne également d'utiliser ces composés pour leurs propriétés anti-inflammatoires et comme stimulants de la croissance cellulaire.

Une sapogénine préférée est la diosgénine, ou spirost-5-èn-3-beta-ol, qui peut être extraite de fenugrec ou de diverses Dioscorées, par exemple de racine d'igname sauvage.

Toutefois, on a constaté que la diosgénine était difficile à solubiliser, et donc à formuler, dans les solvants physiologiquement acceptables pour une application topique sur la peau, en raison de son point de fusion élevé (proche de 204-207°C) et de sa tendance à la recristallisation dans ces solvants.

Ce problème de solubilisation ne se pose pas dans le cas des saponosides, ou saponines, en raison de la présence d'un groupement osidique hydrophile dans la structure de ces composés. Ce groupement osidique confère aux saponines un caractère polaire qui, associé à la partie apolaire constitué par la sapogénine auquel il est fixé, fait de ces composés des émulsionnants naturels. Cette propriété des saponines est notamment décrite dans les documents GB-A-2 080 142 et FR-A-2 466 273, dans lesquelles ces saponines sont introduites dans des émulsions renfermant notamment des huiles végétales et éventuellement des filtres solaires lipophiles ou des alcools gras. Le but de la présente invention est donc de proposer un moyen physiologiquement acceptable qui permette de solubiliser la diosgénine et les autres sapogénines de structure similaire, en une quantité suffisante pour obtenir l'effet recherché et pendant une période de temps suffisante à température ambiante pour assurer une durée de conservation correcte de la composition renfermant la sapogénine.

Or, la Demanderesse a découvert que l'utilisation d'un système solubilisant particulier permettait d'atteindre ce but.

La présente invention a donc pour objet une composition comprenant, dans un milieu physiologiquement acceptable, au moins une sapogénine et un système solubilisant choisi parmi :
(a) (i) au moins un ester non émulsifiant d'acide gras et/ou d'alcool gras dont la chaîne hydrocarbonée renferme au moins 8 atomes de carbone, ou une huile végétale, et (ii) au moins un alcool gras ramifié dont la chaîne hydrocarbonée renferme au moins 8 atomes de carbone ;
(b) (i) au moins un ester non émulsifiant d'acide gras et/ou d'alcool gras dont la chaîne hydrocarbonée renferme au moins 8 atomes de carbone, ou une huile végétale, et (ii) au moins un filtre UV lipophile ; et
(c) (i) au moins un ester non émulsifiant d'acide gras et/ou d'alcool gras dont la chaîne hydrocarbonée renferme au moins 8 atomes de carbone, ou une huile végétale, (ii) au moins un alcool gras ramifié dont la chaîne hydrocarbonée renferme au moins 8 atomes de carbone, et (iii) au moins un filtre UV lipophile.

Les inventeurs ont démontré que, si les esters émulsifiants tels que les polysorbates ne solubilisaient pas les sapogénines, certains esters non émulsifiants d'alcool gras et/ou d'acide gras, tels que le benzoate d'alkyle en C₁₂₋₁₅ ou l'huile de ricin permettait de solubiliser à eux seuls une quantité de sapogénine allant jusqu'à 3% en poids, par rapport au poids total de la sapogénine et du solvant. Toutefois, la solution obtenue recristallisait après trois jours. Il a en outre été démontré qu'une quantité de sapogénine limitée à 2% pouvait être solubilisée, sans recristallisation pendant au moins sept jours, dans certains alcools gras ramifiés, tels que l'octyldodécanol, et dans certains filtres UV lipophiles, dont l'octyl méthoxycinnamate.

Ces premiers résultats étaient cependant difficilement exploitables pour une utilisation commerciale des sapogénines. Par ailleurs, bien que les filtres UV lipophiles et les alcools gras soient de bons solvants des sapogénines en eux-mêmes, leur emploi en grande quantité dans des produits pour application topique sur la peau est déconseillé. Les filtres sont en effet relativement coûteux. En outre, ils ont une forte influence sur la rhéologie des compositions les contenant, de sorte qu'ils restreignent la liberté de formulation de ces compositions en terme de texture. De leur côté, les alcools gras ont tendance à irriter et dessécher la peau.

La présente invention repose sur la découverte que certaines associations de deux de ces composés solubilisants offrent une synergie dans la solubilisation de la sapogénine, en ce sens que l'un au moins des effets suivants est obtenu (par rapport aux composés pris individuellement) :
- la température de solubilisation est abaissée,
- la cristallisation est retardée, voire empêchée, et
- le taux de sapogénine solubilisé est augmenté.

Cet effet de synergie a ainsi été mis en évidence pour l'association d'une huile végétale ou d'un ester non émulsifiant d'alcool gras et/ou d'acide gras avec un alcool gras ramifié et pour l'association de cet ester avec un filtre UV lipophile. Ces associations présentent en outre l'avantage de réduire la quantité d'alcool gras ou de filtre nécessaire à la solubilisation de la diosgénine, en remplaçant une partie de ces composés par un ester qui offre au formulateur toute latitude quant au choix du type de texture envisageable pour la composition. L'ester non émulsifiant d'alcool gras et/ou d'acide gras est en outre doté de bonnes propriétés émollientes et présente l'avantage d'être relativement bon marché.

Il est donc nécessaire et suffisant que la composition selon l'invention contienne au moins l'un des couples de composés mentionné précédemment. Toutefois, pour des raisons cosmétiques, il sera parfois avantageux d'avoir recours à un mélange ternaire de ces trois solubilisants.

Ainsi, dans une forme d'exécution préférée, la composition selon l'invention renferme un système solubilisant comprenant : (a) au moins un- ester non émulsifiant d'acide gras et/ou d'alcool gras dont la chaîne hydrocarbonée renferme au moins 8 atomes de carbone, ou une huile végétale, (b) au moins un alcool gras ramifié dont la chaîne hydrocarbonée renferme au moins 8 atomes de carbone et (c) au moins un filtre UV lipophile.

La sapogénine peut être choisie parmi : la diosgénine, l'hécogénine, la smilagénine, la sarsapogénine, la tigogénine, la yamogénine et la yuccagénine.

La présente invention concerne toutefois plus particulièrement la diosgénine. Celle-ci peut être extraite des tubercules de Dioscorées par un procédé comprenant successivement : l'hydrolyse des hétérosides en milieu acide minéral (éventuellement après fermentation et séchage des tubercules) ; et la filtration de la fraction insoluble, qui est ensuite neutralisée, lavée et traitée par un solvant apolaire. D'autres procédés d'extraction sont cependant utilisables. La diosgénine est également disponible dans le commerce auprès de la société SIGMA sous la dénomination commerciale Diosgenin.

La sapogénine peut représenter de 0,001 à 10%, et de préférence de 0,05 à 5%, du poids total de la composition selon l'invention.

Les constituants du système solubilisant selon l'invention seront maintenant décrits plus en détail.

### Ester non émulsifiant d'acide gras et/ou d'alcool gras

L'ester formant le premier composant du système solubilisant selon l'invention peut être un mono-, un di- ou un triester obtenu à partir d'un acide gras dont la chaîne hydrocarbonée renferme au moins 8 atomes de carbone et/ou d'un alcool gras dont la chaîne hydrocarbonée renferme au moins 8 atomes de carbone. Cet ester ne présente pas de propriétés émulsifiantes, c'est-à-dire qu'il n'est généralement pas porteur de groupements polaires tels que des groupes oxyalkylénés ou des fonctions sulfate ou phosphate, par exemple, susceptibles de lui conférer un caractère amphiphile.

Comme monoesters, on utilise de préférence un monoester d'alcool gras ramifié et/ou d'acide gras ramifié. Il est encore plus avantageux que l'acide gras et l'alcool gras soient tous deux ramifiés. Des exemples sont constitués par : l'isononanoate d'isononyle, le néopentanoate d'isodécyle, le néopentanoate d'octyldodécyle, l'isostéarate d'isostéaryle, et le stéarate d'isocétyle. En variante, cependant, la chaîne grasse de l'alcool et/ou de l'acide formant le monoester selon l'invention peut être linéaire, comme dans le cas du myristate d'isopropyle.

Comme diesters, on peut utiliser des diesters d'acide monocarboxylique et de diol, tels que le dicaprylate de propylène glycol et le di-isostéarate de propylène glycol.

Comme triesters, on utilise avantageusement les triesters d'acide gras monocarboxylique et de glycérol. A titre d'exemple, on peut citer le tri-isostéarate de glycéryle ou les triglycérides caprylique/caprique.

En variante, l'ester non émulsifiant d'acide gras et/ou d'alcool gras selon l'invention peut être remplacé par une huile végétale telle que l'huile d'amande d'abricot, l'huile de ricin, l'huile de tournesol, l'huile d'arachide, l'huile de pépins de raisin, l'huile de noix, etc.

L'ester ou l'huile végétale selon l'invention peuvent être utilisés à raison de 0,1 à 80% en poids, de préférence de 1 à 50% en poids et, mieux, de 5 à 15% en poids, par rapport au poids total de la composition.

### Alcool gras ramifié

L'alcool gras ramifié constituant l'un des composés utilisables comme second composant du système solubilisant selon l'invention est tel que sa chaîne hydrocarbonée renferme au moins 8 atomes de carbone. Il s'agit de préférence un alcool de Guerbet ou 2-alkyl alcanol.

Des exemples d'alcools de Guerbet utilisables sont : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol et le cétylarachidol. On utilise de préférence l'hexyldécanol.

L'alcool gras selon l'invention représente habituellement de 0,1 à 30%, de préférence de 1 à 25% et, mieux, de 1 à 20% du poids total de la composition.

### Filtre UV lipophile

Le second composant du système solubilisant selon l'invention peut être constitué, au lieu ou en plus de l'alcool gras ci-dessus, d'un filtre UV lipophile.

Comme filtres solaires lipophiles convenant à une mise en oeuvre dans la présente invention, on peut notamment citer : les dérivés d'acide p-aminobenzoïque, tels que les esters, sels ou amides d'acide p-aminobenzoïque ; les dérivés d'acide salicylique tels que les esters ou sels d'acide salicylique ; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'acide phénylbenzimidazole sulfonique et ses sels ; l'acide urocanique ou son ester éthylique ; les benzotriazoles ; les dérivés d'hydroxyphényltriazine ; les bis-résorcinol-dialkylaminotriazine ; et leurs mélanges.

Le filtre solaire lipophile selon l'invention est de préférence choisi parmi : l'octyl salicylate ; la benzophénone-3 ; le butyl méthoxydibenzoylméthane ; l'octocrylène ; l'octyl méthoxycinnamate et le composé de formule (II) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl] phénol, décrit dans la demande de brevet EP-A-0 392 883 :

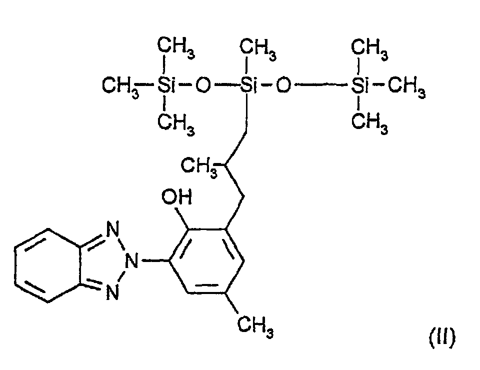

La quantité de filtre UV lipophile utilisé dans la présente invention dépend non seulement de la quantité de sapogénine à solubiliser et des quantités des autres co-solvants ci-dessus, mais est également fonction du Facteur de Protection Solaire (SPF) que l'on souhaite conférer à la composition. Le filtre peut ainsi représenter de 0,001 à 30% du poids total de la composition. Dans le cas où la composition est destinée au soin quotidien de la peau, on utilise de préférence une quantité de filtre UV représentant de 1 à 10% du poids total de la composition.

La composition selon l'invention peut se présenter sous toutes les formes galéniques normalement utilisées pour une application topique sur la peau, notamment sous forme d'une solution huileuse, d'une émulsion huile-dans-eau ou eau-dans-huile ou multiple, d'une émulsion siliconée, d'une microémulsion ou nanoémulsion, d'un gel huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau, ou comme produit capillaire, par exemple comme shampooing ou après-shampooing.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 80 % en poids, et de préférence de 5 à 50 % en poids par rapport au poids total de la composition.

Comme matières grasses utilisables dans l'invention, on peut utiliser, outre le système solubilisant défini précédemment, les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone. L'utilisation de polyisobutène hydrogéné est toutefois déconseillée, de même que l'huile de paraffine et la vaseline, ces composés ayant tendance à agir comme contre-solvants vis-à-vis de la sapogénine.

Les émulsionnants et les coémulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 30 % en poids, et de préférence de 0,5 à 20 % en poids par rapport au poids total de la composition. Comme émulsionnants et coémulsionnants utilisables dans l'invention, dans lequel il est particulièrement avantageux d'utiliser les esters d'acide gras et de polyol tels que le stéarate de PEG-100, le stéarate de PEG-50 et le stéarate de PEG-40 ; le tristéarate de sorbitane, les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les dépigmentants, les émollients, les hydratants, les anti-séborrhéiques, les anti-acnéiques, les agents favorisant la repousse des cheveux, les agents kératolytiques et/ou desquamants, les agents anti-rides, les agents anti-irritants, les agents apaisants et leurs mélanges.

En cas d'incompatibilité entre eux ou avec la sapogénine, les actifs indiqués ci-dessus et/ou la sapogénine peuvent être incorporés dans des sphérules, notamment des vésicules ioniques ou non-ioniques et/ou des nanoparticules (nanocapsules et/ou nanosphères), de manière à les isoler les uns des autres dans la composition.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée. En particulier, ces composés ne devront pas nuire aux propriétés avantageuses de la sapogénine, ni favoriser sa recristallisation.

La présente invention concerne également un procédé de solubilisation d'une sapogénine, caractérisé en ce qu'il comprend l'étape consistant à mélanger la sapogénine à un système solubilisant choisi parmi :
(a) (i) au moins un ester non émulsifiant d'acide gras et/ou d'alcool gras dont la chaîne hydrocarbonée renferme au moins 8 atomes de carbone, ou une huile végétale, et (ii) au moins un alcool gras ramifié dont la chaîne hydrocarbonée renferme au moins 8 atomes de carbone ;
(b) (i) au moins un ester non émulsifiant d'acide gras et/ou d'alcool gras dont la chaîne hydrocarbonée renferme au moins 8 atomes de carbone, ou une huile végétale, et (ii) au moins un filtre UV lipophile ; et
(c) (i) au moins un ester non émulsifiant d'acide gras et/ou d'alcool gras dont la chaîne hydrocarbonée renferme au moins 8 atomes de carbone, ou une huile végétale, (ii) au moins un alcool gras ramifié dont la chaîne hydrocarbonée renferme au moins 8 atomes de carbone, et (iii) au moins un filtre UV lipophile.

Le mélange de la sapogénine et du système solubilisant se fait généralement à chaud, par exemple à une température voisine de 70°C.

L'invention sera maintenant illustrée à l'aide des exemples non limitatifs suivants.

### EXEMPLES

### Exemple 1 : solubilisation dans un mélange triester d'acide gras ramifié et de polyol / alcool gras ramifié

On a réalisé trois compositions comprenant chacune 2% en poids de diosgénine fournie par SIGMA dans un solvant distinct, à savoir :
Pour la composition 1A : le tri-isostéarate de triglycérol disponible auprès de STEARINERIE DUBOIS sous cette dénomination commerciale
Pour la composition 1B : l'hexyldécanol disponible auprès de CONDEA sous la dénomination commerciale ISOFOL 16
Pour la composition 1C : un mélange à 50/50 (en poids) de tri-isostéarate de triglycérol et d'hexyldécanol

Les compositions ci-dessus ont été préparées de la manière suivante. On a pesé et placé dans un pilulier hermétique 100 mg de diosgénine, auquel 4,9 g du ou des solvant(s) ont été ajoutés sous agitation magnétique à 25°C pendant une heure au plus. Dans le cas où la diosgénine ne s'était pas dissoute au bout d'une heure, la suspension a été chauffée sous agitation à 50°C au bain-marie puis, à défaut de solubilisation de la diosgénine après une heure à cette température, la température de la suspension a été portée à 70°C.

Les résultats obtenus sont rassemblés dans le Tableau 1 ci-dessous.

**Tableau 1**

| Composition | Température de solubilisation | Retard à la cristallisation |
|---|---|---|
| 1A | Insoluble | Insoluble |
| 1B | 70°C | 3 jours |
| 1C | 50°C | > 15 jours* |

| | | |
|---|---|---|
| * Durée de l'expérience. | | |

Le mode opératoire ci-dessus a été répété en faisant varier la quantité de diosgénine mise en solution. On a ainsi montré que la quantité maximale de diosgénine pouvant être solubilisée à 50°C dans le mélange de solvants était de 2%, alors qu'elle n'était que de 1% dans chacun des solvants pris individuellement.

Il résulte de ce qui précède que le mélange d'ester non émulsifiant et d'alcool gras ramifié permet de potentialiser les effets de ces composés sur la solubilisation de la diosgénine.

### Exemple 2 : solubilisation dans un mélange monoester d'acide gras linéaire et d'alcool gras ramifié / alcool gras ramifié

De la même manière que dans l'Exemple 1, on a réalisé trois compositions comprenant chacune 2% en poids de diosgénine fournie par SIGMA dans un solvant distinct, à savoir
Pour la composition 2A : le myristate d'isopropyle disponible auprès de STEARINERIE DUBOIS sous cette dénomination commerciale
Pour la composition 2B : l'hexyldécanol disponible auprès de CONDEA sous la dénomination commerciale ISOFOL 16
Pour la composition 2C : un mélange à 50/50 (en poids) de myristate d'isopropyle et d'hexyldécanol

Les résultats obtenus sont rassemblés dans le Tableau 2 ci-dessous.

**Tableau 2**

| Composition | Température de solubilisation | Retard à la cristallisation |
|---|---|---|
| 2A | 70°C | 2 jours |
| 2B | 70°C | 3 jours |
| 2C | 25°C | > 15 jours* |

| | | |
|---|---|---|
| * Durée de l'expérience. | | |

Le mode opératoire ci-dessus a été répété en faisant varier la quantité de diosgénine mise en solution. On a ainsi montré que la quantité maximale de diosgénine pouvant être solubilisée à 50°C dans le mélange de solvants était de 4%, alors qu'elle n'était que de 1% dans chacun des solvants pris individuellement.

Il résulte de ce qui précède que le mélange d'ester non émulsifiant et d'alcool gras ramifié permet de potentialiser les effets de ces composés sur la solubilisation de la diosgénine.

### Exemple 3 : solubilisation dans un mélange monoester d'alcool gras et d'acide gras ramifiés / alcool gras ramifié

De la même manière que dans l'Exemple 1, on a réalisé trois compositions comprenant chacune 2% en poids de diosgénine fournie par SIGMA dans un solvant distinct, à savoir :
Pour la composition 3A : l'isononanoate d'isononyle disponible auprès de STEARINERIE DUBOIS sous cette dénomination commerciale
Pour la composition 3B : l'hexyldécanol disponible auprès de CONDEA sous la dénomination commerciale ISOFOL 16
Pour la composition 3C : un mélange à 50/50 (en poids) d'isononanoate d'isononyle et d'hexyldécanol

Les résultats obtenus sont rassemblés dans le Tableau 3 ci-dessous.

**Tableau 3**

| Composition | Température de solubilisation | Retard à la cristallisation |
|---|---|---|
| 3A | 70°C | 5 heures |
| 3B | 70°C | 3 jours |
| 3C | 40°C | > 15 jours* |

| | | |
|---|---|---|
| * Durée de l'expérience. | | |

Le mode opératoire ci-dessus a été répété en faisant varier la quantité de diosgénine mise en solution. On a ainsi montré que la quantité maximale de diosgénine pouvant être solubilisée à 50°C dans le mélange de solvants était de 3%, alors qu'elle n'était que de 1% dans chacun des solvants pris individuellement.

Il résulte de ce qui précède que le mélange d'ester non émulsifiant et d'alcool gras ramifié permet de potentialiser les effets de ces composés sur la solubilisation de la diosgénine.

### Exemple 4 : solubilisation dans un mélange monoester d'acide gras et d'alcool gras ramifiés / filtre UV lipophile

De la même manière que dans l'Exemple 1, on a réalisé trois compositions comprenant chacune 2% en poids de diosgénine fournie par SIGMA dans un solvant distinct, à savoir :
Pour la composition 4A : l'isononanoate d'isononyle disponible auprès de STEARINERIE DUBOIS sous cette dénomination commerciale
Pour la composition 4B : l'octyl méthoxycinnamate disponible auprès de GIVAUDAN sous la dénomination commerciale PARSOL MCX
Pour la composition 4C : un mélange à 50/50 (en poids) d'isononanoate d'isononyle et d'octyl méthoxycinnamate

Les résultats obtenus sont rassemblés dans le Tableau 4 ci-dessous.

**Tableau 4**

| Composition | Température de solubilisation | Retard à la cristallisation |
|---|---|---|
| 4A | 70°C | 5 heures |
| 4B | 50°C | 7 jours |
| 4C | 50°C | 7 jours |

On a ensuite fait varier la quantité de diosgénine mise en solution. On a ainsi montré que la quantité maximale de diosgénine pouvant être solubilisée à 50°C dans le mélange de solvants était de 3%, alors qu'elle n'était que de 1% dans l'ester et de 2% dans le filtre UV.

Il résulte de ce qui précède que le mélange d'ester et de filtre permet de potentialiser les effets de ces composés sur la quantité de diosgénine susceptible d'être solubilisée dans ces solvants. En outre, la solubilisation de 2% de diosgénine se fait aussi bien dans le mélange de solvants que dans le filtre seul, ce qui permet d'utiliser ce demier à des doses moins élevées et d'améliorer ainsi les qualités cosmétiques de la composition et son coût de fabrication.

### Exemple 5 : Composition cosmétique

On a préparé la composition suivante :

| *Phase A1* | |
|---|---|
| Eau déminéralisée | 58,30 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isononyle | 11,50 % |
| Octyldodécanol | 15,00 % |
| Diosgénine | 0,50 % |
| Butyl hydroxytoluène | 0,10 % |
| Octyl méthoxycinnamate | 1,00 % |
| Conservateurs | 0,15 % |

| *Phase C* | |
|---|---|
| Eau déminéralisée | 2,00 % |
| Triéthanolamine | 0,30 % |

| Phase D | |
|---|---|
| Eau déminéralisée | 1,50 % |
| Conservateur | 0,30 % |

Les constituants de la phase A1 sont mélangés à 70°C, sous agitation. Les constituants des phases A2 et B sont mélangés et chauffés à 70°C au bain-marie, puis le mélange est émulsionné dans la phase A1 à la même température, sous agitation à 600 tours/mn, pendant environ deux minutes. La phase C est préparée à température ambiante, par dissolution dans l'eau de la triéthanolamine, sous agitation magnétique, puis elle est ajoutée à environ 800 tours/mn dans le mélange précédent que l'on laisse ensuite refroidir. La phase D est préparée de la même manière que la phase C et ajoutée au mélange refroidi en-dessous de 50°C. On laisse ensuite refroidir la composition jusqu'à température ambiante.

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 6 : composition cosmétique

De la même manière que dans l'Exemple 5, on a préparé la composition suivante :

| *Phase A1* | |
|---|---|
| Eau déminéralisée | 57,80 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isononyle | 11,00 % |
| Octyldodécanol | 15,00 % |
| Diosgénine | 0,50 % |
| Butyl hydroxytoluène | 0,10 % |
| Octocrylène | 2,00 % |
| Conservateurs | 0,15 % |

| Phase C | |
|---|---|
| Eau déminéralisée | 2,00 % |
| Triéthanolamine | 0,30 % |

| Phase D | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 7 : composition cosmétique

De la même manière que dans l'Exemple 5, on a préparé la composition suivante :

| *Phase A1* | |
|---|---|
| Eau déminéralisée | 66,70 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | - 2,10 % |
| Alcool cétylique | 2,60 % |
| Isononanoate d'isononyle | 6,00 % |
| Octyldodécanol | 7,50 % |
| Diosgénine | 0,10 % |
| Butyl hydroxytoluène | 0,10 % |
| Triglycérides caprylique /caprique | 6,00 % |
| Conservateurs | 0,15 % |

| *Phase C* | |
|---|---|
| Eau déminéralisée | 2,00 % |
| Triéthanolamine | 0,30 % |

| *Phase D* | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 8 : composition cosmétique

De la même manière que dans l'Exemple 5, on a préparé la composition suivante :

| *Phase A1* | |
|---|---|
| Eau déminéralisée | 66,70 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isononyle | 6,00 % |
| Octyldodécanol | 7,50 % |
| Diosgénine | 0,10 % |
| Butyl hydroxytoluène | 0,10 % |
| Huile d'amande d'abricot | 6,00 % |
| Conservateurs | 0,15 % |

| *Phase C* | |
|---|---|
| Eau déminéralisée | 2,00 % |
| Triéthanolamine | 0,30 % |

| *Phase D* | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 9 : composition cosmétique

De la même manière que dans l'Exemple 5, on a préparé la composition suivante :

| *Phase A1* | |
|---|---|
| Eau déminéralisée | 60,80 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isononyle | 9,00 % |
| Octyldodécanol | 10,00 % |
| Diosgénine | 0,50 % |
| Butyl hydroxytoluène | 0,10 % |
| Octyl méthoxycinnamate | 6,00 % |
| Conservateurs | 0,15 % |

| *Phase C* | |
|---|---|
| Eau déminéralisée | 2,00 % |
| Triéthanolamine | 0,30 % |

| *Phase D* | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 10 : composition cosmétique

De la même manière que dans l'Exemple 5, on a préparé la composition suivante :

| *Phase A1* | |
|---|---|
| Eau déminéralisée | 58,80 % |
| Conservateur | 0,25 % |
| Carbomer | 0,40 % |
| Glycérine | 3,00 % |
| Gomme de xanthane | 0,10 % |

| *Phase A2* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isononyle | 7,00 % |
| Octyldodécanol | 10,00 % |
| Diosgénine | 0,50 % |
| Butyl hydroxytoluène | 0,10 % |
| Octocrylène | 10,00 % |
| Conservateurs | 0,15 % |

| *Phase C* | |
|---|---|
| Eau déminéralisée | 2,00 % |
| Triéthanolamine | 0,30 % |

| *Phase D* | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

### Exemple 11 : composition cosmétique

De la même manière que dans l'Exemple 5, on a préparé la composition suivante :

| *Phase A1* | |
|---|---|
| Eau déminéralisée | 63,30 % |
| Conservateur | 0,25 % |
| Carbomer | 0,40 % |
| Glycérine | 3,00 % |
| Gomme de xanthane | 0,10 % |

| *Phase A3* | |
|---|---|
| Stéarate de sorbitan oxyéthyléné 20 OE | |
| (Polysorbate 60) | 0,90 % |

| *Phase B* | |
|---|---|
| PEG-100 stéarate et glycéryl stéarate | 2,10 % |
| Alcool cétylique | 2,60 % |
| Isononanoate d'isonanyle | 11,50 % |
| Octyldodécanol | 10,00 % |
| Diosgénine | 0,50 % |
| Butyl hydroxytoluène | 0,10 % |
| Octyl méthoxycinnamate | 1,00 % |
| Conservateurs | 0,15 % |

| *Phase C* | |
|---|---|
| Eau déminéralisée | 2,00 % |
| Triéthanolamine | 0,30 % |

| *Phase D* | |
|---|---|
| Eau déminéralisée | 1,50 % |
| Conservateur | 0,30 % |

Une observation microscopique de la composition obtenue montre que l'émulsion est fine, régulière, à bords nets, et qu'elle ne présente pas de cristaux. En outre, l'analyse physico-chimique de la composition montre qu'elle est stable après deux mois de conservation à 4°C, 25°C et 45°C. En particulier, on ne constate pas de recristallisation de la diosgénine après cette période de temps, aux températures indiquées. En outre, la stabilité chimique de la diosgénine, telle que déterminée par HPLC, n'est pas affectée.

## Revendications

1. Composition comprenant, dans un milieu physiologiquement acceptable, au moins une sapogénine et un système solubilisant choisi parmi :
(a) (i) au moins un ester non émulsifiant d'acide gras et/ou d'alcool gras dont la chaîne hydrocarbonée renferme au moins 8 atomes de carbone, ou une huile végétale, et (ii) au moins un alcool gras ramifié dont la chaîne hydrocarbonée renferme au moins 8 atomes de carbone ;
(b) (i) au moins un ester non émulsifiant d'acide gras et/ou d'alcool gras dont la chaîne hydrocarbonée renferme au moins 8 atomes de carbone, ou une huile végétale, et (ii) au moins un filtre UV lipophile ; et
(c) (i) au moins un ester non émulsifiant d'acide gras et/ou d'alcool gras dont la chaîne hydrocarbonée renferme au moins 8 atomes de carbone, ou une huile végétale, (ii) au moins un alcool gras ramifié dont la chaîne hydrocarbonée renferme au moins 8 atomes de carbone, et (iii) au moins un filtre UV lipophile.

2. Composition selon la revendication 1, **caractérisée en ce que** ledit ester est un monoester d'alcool gras ramifié et/ou d'acide gras ramifié.

3. Composition selon la revendication 2, **caractérisée en ce que** ledit ester est choisi parmi : l'isononanoate d'isononyle, le néopentanoate d'isodécyle, le néopentanoate d'octyldodécyle, d'isostéarate d'isostéaryle et le stéarate d'isocétyle.

4. Composition selon la revendication 1, **caractérisée en ce que** ledit ester est un triester d'acide gras monocarboxylique et de glycérol.

5. Composition selon l'une quelconque des revendications 1 à 4, **caractérisée en ce que** ledit ester représente de 5 à 15% du poids total de ladite composition.

6. Composition selon l'une quelconque des revendications 1 à 5, **caractérisée en ce que** ledit alcool gras ramifié est un alcool de Guerbet.

7. Composition selon la revendication 6, **caractérisée en ce que** ledit alcool de Guerbet est choisi parmi : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol et le cétylarachidol.

8. Composition selon l'une quelconque des revendications 1 à 7, **caractérisée en ce que** ledit alcool gras représente de 0,1 à 30% du poids total de ladite composition.

9. Composition selon l'une quelconque des revendications 1 à 8, **caractérisée en ce que** ledit filtre UV lipophile est choisi parmi : les dérivés d'acide p-aminobenzoïque, tels que les esters, sels ou amides d'acide p-aminobenzoïque ; les dérivés d'acide salicylique tels que les esters ou sels d'acide saticylique ; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine; l'acide phénylbenzimidazole sulfonique et ses sels ; l'acide urocanique ou son ester éthylique ; les benzotriazoles ; les dérivés d'hydroxyphényltriazine ; les bis-résorcinol-dialkylaminotriazine ; et leurs mélanges.

10. Composition selon la revendication 9, **caractérisée en ce que** ledit filtre UV lipophile est choisi parmi : l'octyl salicylate ; la benzophénone-3 ; le butyl méthoxydibenzoylméthane ; l'octocrylène ; l'octyl méthoxycinnamate ; et le composé de formule (II) :

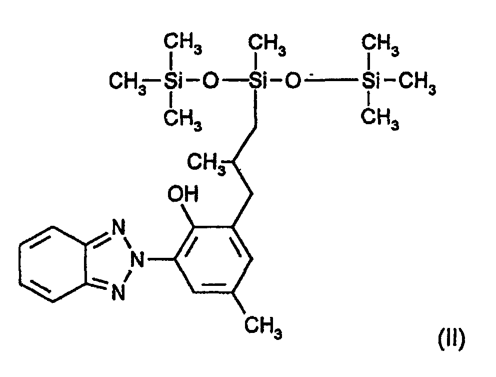

11. Composition selon l'une quelconque des revendications 1 à 10, **caractérisée en ce que** ledit filtre représente de 1 à 10% du poids total de ladite composition.

12. Composition selon l'une quelconque des revendications 1 à 11, **caractérisée en ce qu'**elle renferme un système solubilisant comprenant : au moins un ester non émulsifiant d'acide gras et/ou d'alcool gras dont la chaîne hydrocarbonée renferme au moins 8 atomes de carbone, au moins un alcool gras ramifié dont la chaîne hydrocarbonée renferme au moins 8 atomes de carbone et au moins un filtre UV lipophile.

13. Composition selon l'une quelconque des revendications 1 à 12, **caractérisée en ce que** ladite sapogénine est choisie parmi : la diosgénine, l'hécogénine, la smilagénine, la sarsapogénine, la tigogénine, la yamogénine et la yuccagénine.

14. Composition selon la revendication 13, **caractérisée en ce que** ladite sapogénine est la diosgénine.

15. Composition selon l'une quelconque des revendications 1 à 14, **caractérisée en ce qu'**elle renferme de 0,001 à 10% en poids, et de préférence de 0,05 à 5% en poids, de sapogénine.

16. Procédé de solubilisation d'une sapogénine, **caractérisé en ce qu'**il comprend l'étape consistant à mélanger la sapogénine à un système solubilisant choisi parmi :
(a) (i) au moins un ester non émulsifiant d'acide gras et/ou d'alcool gras dont la chaîne hydrocarbonée renferme au moins 8 atomes de carbone, ou une huile végétale, et (ii) au moins un alcool gras ramifié dont la chaîne hydrocarbonée renferme au moins 8 atomes de carbone ;
(b) (i) au moins un ester non émulsifiant d'acide gras et/ou d'alcool gras dont la chaîne hydrocarbonée renferme au moins 8 atomes de carbone, ou une huile végétale, et (ii) au moins un filtre UV lipophile ; et
(c) (i) au moins un ester non émulsifiant d'acide gras et/ou d'alcool gras dont la chaîne hydrocarbonée renferme au moins 8 atomes de carbone, ou une huile végétale, (ii) au moins un alcool gras ramifié dont la chaîne hydrocarbonée renferme au moins 8 atomes de carbone, et (iii) au moins un filtre UV lipophile.

## Patentansprüche

1. Zusammensetzung, die in einem physiologisch akzeptablen Medium mindestens ein Sapogenin und ein Solubilisierungssystem enthält, das ausgewählt ist unter:
(a) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl und (ii) mindestens einem verzweigten Fettalkohol, dessen Kohlenwasserstoffkette mindestens 8 Kohlenstoffatome aufweist;
(b) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl und (ii) mindestens einem lipophilen UV-Filter; und
(c) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl, (ii) mindestens einem verzweigten Fettalkohol, dessen Kohlenwasserstoffkette mindestens 8 Kohlenstoffatome aufweist, und (iii) mindestens einem lipophilen UV-Filter.

2. Zusammensetzung nach Anspruch 1, **dadurch gekennzeichnet, daß** der Ester ein Monoester eines verzweigten Fettalkohols und/oder einer verzweigten Fettsäure ist.

3. Zusammensetzung nach Anspruch 2, **dadurch gekennzeichnet, daß** der Ester ausgewählt ist unter: Isononylisononanoat, Isodecylneopentanoat, Octyldodecylneopentanoat, Isostearylisostearat und Isocetylstearat.

4. Zusammensetzung nach Anspruch 1, **dadurch gekennzeichnet, daß** der Ester ein Triester einer Monocarbonsäure und von Glycerin ist.

5. Zusammensetzung nach einem der Ansprüche 1 bis 4, **dadurch gekennzeichnet, daß** der Ester 5 bis 15 % des Gesamtgewichts der Zusammensetzung ausmacht.

6. Zusammensetzung nach einem der Ansprüche 1 bis 5, **dadurch gekennzeichnet, daß** der verzweigte Fettalkohol ein Guerbet-Alkohol ist.

7. Zusammensetzung nach Anspruch 6, **dadurch gekennzeichnet, daß** der Guerbet-Alkohol ausgewählt ist unter: Butyloctanol, Hexyldecanol, Octyldecanol, Isostearylalkohol, Octyldodecanol, Decyltetradecanol, Undecylpentadecanol, Dodecylhexadecanol, Tetradecyloctadecanol, Hexyldecyloctadecanol, Tetradecyleicosanol und Cetylarachidol.

8. Zusammensetzung nach einem der Ansprüche 1 bis 7, **dadurch gekennzeichnet; daß** der Fettalkohol 0,1 bis 30 % des Gesamtgewichts der Zusammensetzung ausmacht.

9. Zusammensetzung nach einem der Ansprüche 1 bis 8, **dadurch gekennzeichnet, daß** das lipophile UV-Filter ausgewählt ist unter: p-AmMobenzoesäurederivaten, beispielsweise Estern, Salzen oder Amiden von p-Aminobenzoesäure; Salicylsäurederivaten, beispielsweise Estern oder Salzen von Salicylsäuren; Benzophenonderivaten; Dibenzoylmethanderivaten; Diphenylacrylatderivaten; Benzofuranderivaten; polymeren UV-Filtern, die eine oder mehrere Silicium-organische Gruppen enthalten; Zimtsäureestern; Campherderivaten; Trianilino-s-triazinderivaten; Phenylbenzimidazolsulfonsäuren und ihren Salzen; Urocansäure oder ihrem Ethylester; Benzotriazolen; Hydroxyphenyltriazinderivaten; Bis-Resorcindialkylaminotriazinen; und deren Gemischen.

10. Zusammensetzung nach Anspruch 9, **dadurch gekennzeichnet, daß** das lipophile UV-Filter ausgewählt ist unter: Octylsalicylat; Benzophenon-3; Butylmethoxydibenzoylmethan; Octocrylen; Octylmethoxycinnamat; und der Verbindung der Formel (II):

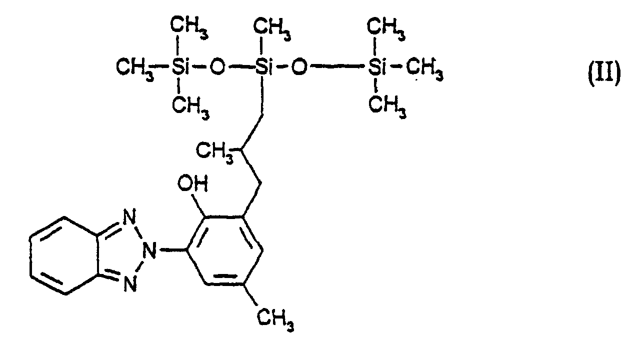

11. Zusammensetzung nach einem der Ansprüche 1 bis 10, **dadurch gekennzeichnet, daß** das Filter 1 bis 10 % des Gesamtgewichts der Zusammensetzung ausmacht.

12. Zusammensetzung nach einem der Ansprüche 1 bis 11, **dadurch gekennzeichnet, daß** sie ein Solubilisierungssystem enthält, mit: mindestens einem nicht emulgierenden Ester einer Fettsäure und/oder eines Fettalkohols, dessen Kohlenwasserstoffkette mindestens 8 Kohlenstoffatome aufweist, mindestens einem verzweigten Fettalkohol, dessen Kohlenwasserstoffkette mindestens 8 Kohlenstoffatome aufweist, und mindestens einem lipophilen UV-Filter.

13. Zusammensetzung nach einem der Ansprüche 1 bis 12, **dadurch gekennzeichnet, daß** das Sapogenin ausgewählt ist unter: Diosgenin, Hecogenin, Smilagenin, Sarsapogenin, Tigogenin, Yamogenin und Yuccagenin.

14. Zusammensetzung nach Anspruch 13, **dadurch gekennzeichnet, daß** das Sapogenin das Diosgenin ist.

15. Zusammensetzung nach einem der Ansprüche 1 bis 14, **dadurch gekennzeichnet, daß** sie 0,001 bis 10 Gew.-% und vorzugsweise 0,05 bis 5 Gew.-% Sapogenin enthält.

16. Verfahren zur Solubilisierung eines Sapogenins, **dadurch gekennzeichnet, daß** es einen Schritt umfaßt, der darin besteht, das Sapogenin mit einem Solubilisierungssystem zu vermischen, das ausgewählt ist unter:
a) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl und (ii) mindestens einem verzweigten Fettalkohol, dessen Kohlenwasserstoffkette mindestens 8 Kohlenstoffatome aufweist;
b) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl und (ii) mindestens einem lipophilen UV-Filter; und
c) (i) mindestens einem nicht emulgierenden Ester einer Fettsäure und/oder eines Fettalkohols, dessen Kohlenwasserstoffkette mindestens 8 Kohlenstoffatome aufweist, oder einem pflanzlichen Öl, (ii) mindestens einem verzweigten Fettalkohol, dessen Kohlenwasserstoffkette mindestens 8 Kohlenstoffatome aufweist, und (iii) mindestens einem lipophilen UV-Filter.

## Claims

1. Composition comprising, in a physiologically acceptable medium, at least one sapogenin and a solubilizing system chosen from:
(a) (i) at least one non-emulsifying ester of a fatty acid and/or of a fatty alcohol whose hydrocarbon-based chain contains at least 8 carbon atoms, or a plant oil, and (ii) at least one branched fatty alcohol whose hydrocarbon-based chain contains at least 8 carbon atoms;
(b) (i) at least one non-emulsifying ester of a fatty acid and/or of a fatty alcohol whose hydrocarbon-based chain contains at least 8 carbon atoms, or a plant oil, and (ii) at least one lipophilic UV screening agent; and
(c) (i) at least one non-emulsifying ester of a fatty acid and/or of a fatty alcohol whose hydrocarbon-based chain contains at least 8 carbon atoms, or a plant oil, (ii) at least one branched fatty alcohol whose hydrocarbon-based chain contains at least 8 carbon atoms, and (iii) at least one lipophilic UV screening agent.

2. Composition according to Claim 1, **characterized in that** the said ester is a monoester of a branched fatty alcohol and/or of a branched fatty acid.

3. Composition according to Claim 2, **characterized in that** the said ester is chosen from:
isononyl isononanoate, isodecyl neopentanoate, octyldodecyl neopentanoate, isostearyl isostearate and isocetyl stearate.

4. Composition according to Claim 1, **characterized in that** the said ester is a triester of a monocarboxylic fatty acid and of glycerol.

5. Composition according to any one of Claims 1 to 4, **characterized in that** the said ester represents from 5% to 15% relative to the total weight of the said composition.

6. Composition according to any one of Claims 1 to 5, **characterized in that** the said branched fatty alcohol is a Guerbet alcohol.

7. Composition according to Claim 6, **characterized in that** the said Guerbet alcohol is chosen from: butyloctanol, hexyldecanol, octyldecanol, isostearyl alcohol, octyldodecanol, decyltetradecanol, undecylpentadecanol, dodecylhexadecanol, tetradecyloctadecanol, hexyldecyloctadecanol, tetradecyleicosanol and cetylarachidol.

8. Composition according to any one of Claims 1 to 7, **characterized in that** the said fatty alcohol represents from 0.1% to 30% relative to the total weight of the said composition.

9. Composition according to any one of Claims 1 to 8, **characterized in that** the said lipophilic UV screening agent is chosen from: p-aminobenzoic acid derivatives, such as p-aminobenzoic acid esters, salts or amides; salicylic acid derivatives such as salicylic acid esters or salts; benzophenone derivatives; dibenzoylmethane derivatives; diphenylacrylate derivatives; benzofuran derivatives; polymeric UV screening agents containing one or more or anosilicon residues; cinnamic acid esters; camphor derivatives; trianilino-s-triazine derivatives; phenylben imidazole-sulphonic acid and its salts; urocanic acid or its ethyl ester; benzotriazoles; hydroxyphenyltriazine derivatives; bis-resorcinol-dialkylaminotriazines; and mixtures thereof.

10. Composition according to Claim 9, **characterized in that** the said lipophilic UV screening agent is chosen from: octyl salicylate; benzophenone-3; butylmethoxydibenzoylmethane; octocrylene; octyl methoxycinnamate; and the compound of formula (II) :

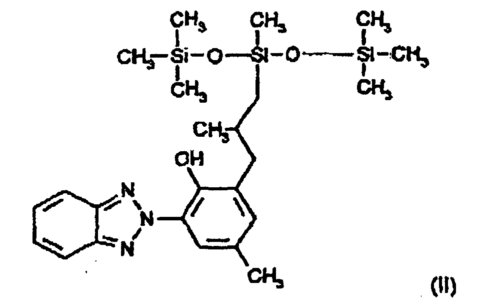

11. Composition according to any one of Claims 1 to 10, **characterized in that** the said screening agent represents from 1% to 10% relative to the total weight of the said composition.

12. Composition according to any one of Claims 1 to 11, **characterized in that** it contains a solubilizing system comprising: at least one non-emulsifying ester of a fatty acid and/or of a fatty alcohol whose hydrocarbon-based chain contains at least 8 carbon atoms, at least one branched fatty alcohol whose hydrocarbon-based chain contains at least 8 carbon atoms, and at least one lipophilic UV screening agent.

13. Composition according to any one of Claims 1 to 12, **characterized in that** the said sapogenin is chosen from: diosgenin, hecogenin, smilagenin, sarsapogenin, tigogenin, yamogenin and yuccagenin.

14. Composition according to Claim 13, **characterized in that** the said sapogenin is diosgenin.

15. Composition according to any one of Claims 1 to 14, **characterized in that** it contains from 0.001% to 10% by weight and preferably from 0.05% to 5% by weight of sapogenin.

16. Process for dissolving a sapogenin, **characterized in that** it comprises the step which consists in mixing the sapogenin with a solubilizing system chosen from:
(a) (i) at least one non-emulsifying ester of a fatty acid and/or of a fatty alcohol whose hydrocarbon-based chain contains at least 8 carbon atoms, or a plant oil, and (ii) at least one branched fatty alcohol whose hydrocarbon-based chain contains at least 8 carbon atoms;
(b) (i) at least one non-emulsifying ester of a fatty acid and/or of a fatty alcohol whose hydrocarbon-based chain contains at least 8 carbon atoms, or a plant oil, and (ii) at least one lipophilic UV screening agent; and
(c) (i) at least one non-emulsifying ester of a fatty acid and/or of a fatty alcohol whose hydrocarbon-based chain contains at least 8 carbon atoms, or a plant oil, (ii) at least one branched fatty alcohol whose hydrocarbon-based chain contains at least 8 carbon atoms, and (iii) at least one lipophilic UV screening agent.
